# EUROPEAN PATENT SPECIFICATION

(11) **EP 2 764 120 B1**
(45) Date of publication and mention of the grant of the patent: **10.06.2020**
(21) Application number: 12780586.9
(22) Date of filing: 05.10.2012
(51) Int. Cl.: C12Q 1/6883, G01N 33/50

(54) **STIMULUS-ELICITED GENOMIC PROFILE MARKERS OF A NEURODEGENERATIVE CONDITION**
DURCH STIMULUS AUSGELÖSTE GENOMISCHE PROFILMARKER FÜR EINE NEURODEGENERATIVE ERKRANKUNG
MARQUEURS DE PROFIL GÉNOMIQUE D'UN ÉTAT NEURODÉGÉNÉRATIF GÉNÉRÉS PAR UN STIMULUS

(30) Priority: 05.10.2011 US 201161543416 P
(43) Date of publication of application: 13.08.2014
(73) Proprietor: Blanchette Rockefeller Neurosciences Institute, Morgantown, WV 26505-3409 (US); Alkon, Daniel L., Bethesda, MD 20817 (US); Khan, Tapan Kumar, Morgantown, WV 26505 (US)
(72) Inventor: ALKON, Daniel, L., Bethesda, MD 20817 (US); KHAN, Tapan, K., Morgantown, WV 26505 (US)
(74) Representative: ABG Intellectual Property Law, S.L.
(86) International application number: PCT/US2012/059137
(87) International publication number: WO 2013/052922

(56) References cited:
- EP-A2- 2 322 191
- WO-A1-97/37228
- WO-A1-2010/014588
- WO-A1-2011/041761
- WO-A2-2008/077094
- KLEINMAN ET AL: "Matrigel: Basement membrane matrix with biological activity", SEMINARS IN CANCER BIOLOGY, SAUNDERS SCIENTIFIC PUBLICATIONS, PHILADELPHIA, PA, US, vol. 15, no. 5, 1 October 2005 (2005-10-01), pages 378-386, XP005021947, ISSN: 1044-579X

## Description

This application claims the benefit of priority under 35 U.S.C. § 119 to United States Provisional Application No. 81/543,418, filed on October 5, 2011.

The present disclosure relates to methods for diagnosing a neurodegenerative condition, such as Alzheimer's disease, using a stimulus-elicited gene expression profile.

Alzheimer's disease (AD) is a neurodegenerative disorder characterized by the progressive decline of memory and cognitive functions, it is estimated that over five million Americans are living with this progressive and fatal disease. Alzheimer's disease destroys brain cells, causing memory loss and problems with thinking and behavior that decrease quality of life. While AD has no known cure, treatments for symptoms can improve the quality of life of the millions of people suffering from AD, and that of their families. An early diagnosis of AD gives the patient time to make choices that maximize qualify of life and to plan for the future, reduces anxiety about unknown problems, and provides a better chance for the patient benefiting from treatment.

The complexity of AD raises a great challenge for early screening. A biological marker that would predict AD prior to symptomatic diagnosis or definitively diagnose early AD could have a major impact in testing and treating AD. The long term prodromal stages, co-morbidity with other non-Alzheimer's disease dementia (non-ADD), and multi-factorial nature of AD offer further challenges for successful diagnosis.

Thus, there exists a need in the art for improved methods of diagnosing Alzheimer's disease.

WO 2010/014588 discloses Stimulus-Elicited Genomic Profile Markers Of Alzheimer's Disease.

WO 2011/041761 discloses Fibroblast Growth Patterns For Diagnosis Of Alzheimer's Disease.

WO 97/37228 discloses Methods For Diagnosing And Treating Alzheimer's Disease.

### BRIEF DESCRIPTION OF THE DRAWINGS

Figure 1 shows the expression level (Microarray data) of tumor necrosis factor receptor superfamily, member 19 (TNFRSF-19) gene in fibroblast cells from subjects with AD (left bar) and in fibroblast cells age-averaged control subjects ("AC") (right bar) at 48 hrs after stimulation with BD Matrigel™.
Figure 2 shows the expression levels (by PGR analysis) of the TNFRSF-19 gene in AD and AC subjects, stimulated by BD Matrigel™ for 48 hours or absent of stimulation by BD Matrigel™.
Figure 3 shows the expression levels (by PGR analysis) of the TNFRSF-19 gene in AD and AC subjects, stimulated by BD Matrigel™ for 48 hours.
Figure 4 shows tissue specific expressions of the TNFRSF-19 gene in normal and cancer cells,

### DESCRIPTION

The present invention provides a method of diagnosing Alzheimer's disease in a human subject in need thereof comprising:
(a) contacting a cell sample from the subject with at least one stimulus, wherein the at least one stimulus is chosen from the group consisting of a polysaccharide mixture comprising at least one basement membrane protein a protein kinase C activator, an Aβ oligomer, or combinations thereof and wherein the cell sample is a fibroblast cell sample or a lymphocyte cell sample; and
(b) detecting the expression of at least one gene in the cell sample, wherein the at least one gene is selected from the group consisting of EFEMP1, BDNF, FGF18, IGFBP5, HAS1, CDH2, CAPG, MMP12, MAPK1, TNFRSF19, PPAPDC1A, DUSP2, CRIP2, PPP1CB, EDNRB, EGR2, MAP2, HLA-C, CADM1, COL23A1, BDKRB2, APOE, and NRG3, wherein
the subject has Alzheimer's disease if the expression level(s) determined in step (b) for one or more gene(s) selected from the group consisting of EFEMP1, BDNF, FGF18, 1GFBP5, HAS1, CDH2, CAPG, MMP12, MAPK1, TNFRSF19, PPAPDC1A, DUSP2, CRIP2, PPP1CB and EDNRB is greater than the expression level(s) of the same gene(s) in age-matched control cells that have been contacted with the same at least one stimulus, and/or
the subject has Alzheimer's disease if the expression level(s) determined in step (b) for one or more gene(s) selected from the group consisting of EGR2, MAP2, HLA-C, CADM1, COL23A1, BDKRB2, APOE and NRG3 is lower than the expression levels(s) of the same gene(s) in age-matched control cells that have been contacted with the same at least one stimulus.

The present disclosure is directed to methods of diagnosing a neurodegenerative condition, e.g., Alzheimer's disease, comprising contacting a cell sample from a subject with at least one stimulus, such as a protein and/or polysaccharide mixture, a protein kinase C activator, an Aβ oligomer (ASPD), an agent, and combinations thereof; and detecting the expression of at least one gene in the cell sample. Methods may further comprise comparing the expression of the at least one gene in the cell sample to the expression of the same at least one gene in control cells; and determining whether the subject has the neurodegenerative condition (Alzheimer's disease), wherein a change in the expression of the at least one gene in the cell sample compared to the expression of the same at least one gene in the control cells indicates that the subject has the neurodegenerative condition (Alzheimer's disease).

Particular aspects of the disclosure are described in greater detail below. The terms and definitions as used in the present application and as clarified herein are intended to represent the meaning within the present disclosure.

The singular forms "a," "an," and "the" include plural reference unless the context dictates otherwise.

The term "neurodegenerative condition" refers to a condition resulting in the progressive loss of structure or function of neurons, including the death of neurons. Conditions may include, but are not limited to, syndromes of progressive dementia such as Alzheimer's disease. Lewy body dementia, amyotrophy;
syndromes of disordered posture and movement, such as Parkinson's disease, multiple symptom atrophy, tourette syndrome; syndromes of progressive ataxia, such as cerebral cortical ataxias; syndromes of slowly developing muscular weakness or atrophy such as amyotrophic lateral sclerosis (ALS); and aging.

The term "Alzheimer's Disease" or "AD" refers to any condition where Aβ and/or neurofibrillary tangles eventually accumulates in the cells of the central nervous system, which accumulation cannot be attributed to other disease or conditions such as CAA. AD may be heritable in a Familial manifestation, or may be Sporadic. As used herein, AD includes Familial, Sporadic, as well as intermediates and subgroups thereof based on phenotypic manifestations. In addition, this term includes the development of Aβ in subjects having Down's Syndrome.

The term "Sporadic AD" refers to AD that develops later in life, usually after the age of about 65, and is not associated with a family history of AD or a mutation in a gene identified as being a risk factor for AD.

The term "young-onset" refers to AD that occurs in a person under age about 65. Young-onset includes but is not limited to Familial AD.

"Familial AD" refers to AD associated with inherited mutations in the presenilin-I gene (PSEN-I), presenilin-2 gene (PSEN-2); the gene encoding Amyloid beta precursor protein (APP), and/or the gene encoding apolipoprotein E (APOE).

"Early-stage AD" refers to the stage of AD associated with moderate symptoms of cognitive decline such as memory loss or confusion. Memory loss or other cognitive deficits are noticeable, yet the person can compensate for them and continue to function independently. This stage correlates with Stage 4 of the Functional Assessment Staging (FAST) scale or mild AD according to the criteria defined in the Diagnostic and Statistical Manual of Mental disorders, 4th Edition (DSM-IV-TR) (published by the American Psychiatric Association), NINCDS-ADRDA, or MMSE.

"Mild Cognitive Impairment (MCI)" refers to a transition stage between the cognitive changes of normal aging and AD. A subject with MCI has cognitive impairments beyond that expected for their age and education, but that do not interfere significantly with their daily activities. A person with MCI may have impairments with memory, language, or another mental function. Not all subjects with MCI develop AD. As used herein, a subject with MCI is considered at risk for developing AD.

Other risk factors for AD are advancing age, mutations in PSEN-I, PSEN-2, APP and APOE.

As used herein, the term "subject" means a mammal. In one embodiment, the subject is a human.

The term "normal subject," as used herein, is relative to the neurodegenerative condition, e.g., AD. That is, the subject does not exhibit AD, is not diagnosed with the specified disease, and is not at risk for developing the disease.

"Peripheral tissue" refers to a tissue that is not derived from neuroectoderm, and specifically includes olfactory epithelium, tongue, skin (including dermis and/or epidermis), and mucosal layers of the body. The term "differentially expressed" or "differential expression" as used herein refers to a measurement of a cellular constituent varies in two samples, a control sample and a test sample. The cellular constituent can be either upregulated in the experiment relative to the control or downregulated in the experiment relative to the control sample.

As used herein, the phrase "detecting the level of expression" includes methods that quantitate expression levels as well as methods that determine whether a gene of interest is expressed at all. The detection can be qualitative or quantitative. In one embodiment, the differential expression is statistically significant.

As used herein, "upregulating" or "upregulation" means detecting an increased the amount or activity of a gene or gene product relative to a baseline or control state, through any mechanism including, but not limited to increased transcription, translation, and/or increased stability of the transcript or protein product. Increased expression in a test cell includes a situation where the corresponding gene in a control cell is either unchanged by stimulation or is downregulated in response to the stimulation.

As used herein, "down regulating" or "downregulation" refers to detecting a decrease in the amount or activity of a gene or gene product relative to a baseline or control state, through any mechanism including, but not limited to decreased transcription, translation, and/or decreased stability of the transcript or protein product. Decreased expression in a test cell includes a situation where the corresponding gene in a control cell is either unchanged by stimulation or is upregulated in response to the stimulation.

A "change in gene expression" refers to detection of upregulation or downregulation.

The term "microarray" or "nucleic acid microarray" refers to a substrate-bound collection of plural nucleic acids, hybridization to each of the plurality of bound nucleic acids being separately detectable. The substrate can be solid or porous, planar or non-planar, unitary or distributed. Microarrays or nucleic acid microarrays include all the devices so called in Schena (ed.), DNA Microarrays: A Practical Approach (Practical Approach Series), Oxford University Press (1999); Nature Genet. 21(1)(suppL):1-60 (1999); Schena (ed.), Microarray Biochip: Tools and Technology, Eaton Publishing Company/BioTechniques Books Division (2000). These microarrays include substrate-bound collections of plural nucleic acids in which the plurality of nucleic acids are disposed on a plurality of beads, rather than on a unitary planar substrate, as is described, inter alia, in Brenner et al., Proc. Natl. Acad. Sci. USA 2000; 97(4):1665-1670.

The terms "about" and "approximately" shall generally mean an acceptable degree of error for the quantity measured given the nature or precision of the measurements. Typical, exemplary degrees of error are within 20 percent (%), preferably within 10%, and more preferably within 5% of a given value or range of values. Alternatively, and particularly in biological systems, the terms "about" and "approximately" may mean values that are within an order of magnitude, preferably within 5-fold and more preferably within 2-fold of a given value. Numerical quantities given herein are approximate unless stated otherwise, meaning that the term "about" or "approximately" can be inferred when not expressly stated.

In one embodiment, the disclosure provides a method of diagnosing a neurodegenerative condition such as AD by detecting differences in the expression levels of genes in cells from a subject suspected of developing or having the neurodegenerative condition (AD) in response to stimulation with at least one stimulus ("a cell sample"), compared to expression of the same genes in normal control cells ("control cells") following stimulation with the same stimulus. In one embodiment, the control cells are derived age-matched control subjects and are stimulated with the same stimulus as the cell sample.

In another embodiment, increased gene expression in the stimulated cell sample compared to the stimulated control cells (upregulation) indicates the presence of the neurodegenerative condition (AD). In another aspect, decreased gene expression in the stimulated cell sample compared to the stimulated control cells (downregulation) indicates the presence of the neurodegenerative condition (AD). In a third aspect, absence of increased gene expression in the stimulated cell sample compared to the stimulated control cells indicates the presence of the neurodegenerative condition (AD). In a fourth aspect, absence of decreased expression in the stimulated cell sample compared to the stimulated control cells indicates the presence of the neurodegenerative condition (AD).

In another embodiment, the present disclosure provides a method for diagnosing early-stage AD by detecting the differential changes in gene expression. In specific embodiments, the method as disclosed herein can be used to distinguish Alzheimer's pathology or dementia from that associated with other forms of dementia, such as frontotemporal degenerative dementias (e.g., Pick's disease, corticobasal ganglionic degenerations, and frontotemporal dementia), Huntington's disease, Creutzfeldt Jakob disease, Parkinson's disease, cerebrovascular disease, head trauma, and substance abuse.

In another embodiment, the disclosure provides a method of evaluating disease progression by applying the methods to two or more samples from the same patient taken on separate occasions. This embodiment can also be used to evaluate the effect of any AD treatment administered after the first sample is taken but before the send sample is taken. Exemplary AD treatments that can be evaluated include Namenda® (memantine), Aricept® (donapazil) and Razadyne® (galantamine), an Exelon® (rivastigmine).

The present disclosure further provides a method of screening therapeutic substances for the treatment or prevention of AD by evaluating the effects of a test agent on the differential expression of genes according to the methods described herein.

In another embodiment, the present disclosure provides kits to carry out the diagnostic method as disclosed herein. Table 1 provides the GenBank accession number for the genes identified to be up-regulated in the AD cells compared with the control cells. Table 2 provides the GenBank accession number for the genes identified to be downregulated in the AD cells compared with the control cells.

For example, TNFRSF-19 or TNFα-19 receptor gene is shown herein to be upregulated in AD cells upon stimulation by BD Matrigel™. TNFRSF-19 (also known as TROY, TAJ, or TRADE) is a TNF family orphan receptor that is expressed in neurons and involved in axon growth. It is a putative membrane-bound protein of 348 amino acids with an extracellular domain and an extended cytoplasmic domain. The gene symbol report of TNFRSF1 9 is listed in Table 4.

TNFRSF-19 is associated with JNK cascade, apoptosis, regulation of I-kappaB kinase/NF-kappaB cascade tumor necrosis factor-mediated signaling pathway. Unlike other TNF receptors, TNFRSF-19 does not appear to play a role in immune response pathways. Thus far, there have been no reports in the literature regarding the relationship between TNFRSF-19 upregulation and AD, demonstrating the potential of the stimulus-elicited genome-wide expression approach to identify new cellular pathways involved in AD.

In another embodiment, the diagnostic method as disclosed herein comprises detecting differential expression in the control sample and the cell sample of at least one gene listed in Table 1 and/or Table 2.

In another embodiment, the diagnostic method as disclosed herein comprises detecting differential expression in the control sample and the cell sample of at least two genes listed in Table 1 and/or Table 2.

In another embodiment, the diagnostic method as disclosed herein comprises detecting differential expression in the control sample and the cell sample of at least five genes listed in Table 1 and/or Table 2.

In another embodiment, the diagnostic method as disclosed herein comprises detecting differential expression in the control sample and the cell sample of at least ten genes listed in Table 1 and/or Table 2.

In another embodiment, the diagnostic method as disclosed herein comprises detecting differential expression in the control sample and the cell sample of at least fifteen genes listed in Table 1 and/or Table 2.

### Biological Samples

The present disclosure provides methods for the diagnosis of a neurodegenerative condition such as Alzheimer's disease using cells from subjects suspected of at risk for developing the neurodegenerative condition (e.g., AD or suspected of having AD). In the methods as disclosed herein, the cells that are taken from the subject include any viable cells. In one embodiment, the cells are from peripheral tissues, *i.e.,* non-neural tissue. In further embodiments, the tissue is from skin, blood, mucosa, or cerebrospinal fluid.

In another embodiment, the cells are fibroblasts, epithethial cells, endothelial cells, or hematopoietic cells including lymphocytes. In a further embodiment, the cells are skin epithelial cells, skin fibroblast cells, blood cells or buccal mucosa cells. The cells may be fresh, cultured, or frozen prior to analysis. In one embodiment, a punch skin biopsy can be used to obtain skin fibroblasts from a subject. Skin fibroblast samples may also be obtained from a subject by using a surgical blade. These fibroblasts are analyzed directly or introduced into cell culture conditions. In another embodiment, the cells are isolated from excised cells using laser capture microdissection to obtain a homogenous population of cells of the same type.

### Stimulus

In some embodiments, the at least one stimulus as disclosed herein is chosen from a protein mixture, a polysaccharide mixture, a protein kinase C (PKC) activator, an Aβ oligomer (ASPD), an agent, and combinations thereof. In an embodiment, the at least one stimulus comprises two or more stimuli, wherein the two or more stimuli are contacted with the cell sample simultaneously or sequentially.

The at least one stimulus comprises a protein and/or polysaccharide mixture, such as a gelatinous protein and/or polysaccharide mixture. For example, stimulation can be induced by culturing the AD cells, AC cells, or non-ADD cells in the protein and/or polysaccharide mixture that induces AD-specific differential gene expression.

In some embodiments, the protein and/or polysaccharide mixture is chosen from laminin, collagen, entactin, heparin sulfate proteoglycan, entactinlnidogen, matrix metalloproteinase, plasminogen activator, growth factor, and any combination thereof. In some embodiments, the protein and/or polysaccharide mixture comprises at least one basement membrane protein.

In some embodiments, the protein and/or polysaccharide mixture comprises a preparation. In some embodiments, the preparation is solubilized. In at least one embodiment, the preparation is extracted from tumor or cancer cells, such as the Engelbreth-Holm-Swarm (EHS) mouse sarcoma, and is rich in extracellular matrix (ECM) proteins. Such preparations may, for example, comprise at least one of laminin, collagen IV, heparan sulfate proteoglycans, and entactin/nidogen. A nonlimiting example suitable for the present disclosure is BD Matrigel™, which is the trade name (BD Biosciences) for a gelatinous protein mixture secreted by EHS mouse sarcoma cells. For example, the components of BD Matrigel™ are listed in Table 3. This mixture resembles the complex extracellular environment found in many tissues, and may be used as a substrate for cell culture. BD Matrigel™ comprises laminin, collagen IV, heparan sulfate proteoglycans, and entactin 1. At 37°C, BD Matrigel™ polymerizes to produce biologically active matrix material resembling the mammalian cellular basement membrane.

In some embodiments of the present disclosure, the preparation further comprises TGF-beta, epidermal growth factor, insulin-like growth factor, fibroblast growth factor, tissue plasminogen activator, and/or other growth factors that may or may not occur naturally in a tumor. In some embodiments, TGF-beta, epidermal growth factor, insulin-like growth factor, fibroblast growth factor, tissue plasminogen activator, and/or other growth factors occur naturally in a tumor, such as the EHS mouse sarcoma tumor. BD Matrigel™ Matrix Growth Factor Reduced (GFR), for example, may be suitable for applications requiring a more highly defined basement membrane preparation of the gel substrate. In some embodiments, the at least one stimulus is a more defined basement membrane preparation than BD Matrigel™ Matrix Growth Factor Reduced.

The preparation may comprise an ECM protein preparation effective for the attachment and differentiation of both normal and transformed anchorage dependent epithelial and other cell types. Exemplary cell types include, but are not limited to, neurons, hepatocytes, Sertoli cells, chick lens, and vascular endothelial cells. The ECM protein preparation may influence gene expression in adult rat hepatocytes as well as three-dimensional culture in mouse and human mammary epithelial cells. The preparation may, for example, serve as the basis for tumor cell invasion assays, support *in vivo* peripheral nerve regeneration, and/or provide a substrate for the study of angiogenesis both *in vitro* and *in vivo.* The ECM protein may also support *in vivo* propagation of human tumors in immunosupressed mice.

In some embodiments of the present disclosure, a volume of chilled ECM protein is dispensed onto tissue culture labware. As used herein, the term "chilled" refers to a temperature less than room temperature, for example, less than about 15°C, less than about 10°C, less than about 5°C, e.g., a temperature of about 4°C. When incubated at an elevated temperature, the ECM proteins may self-assemble to produce a thin film that covers the surface of the labware. As used herein, the term "elevated" refers to a temperature above room temperature, such as above about 20°C, above about 25°C, above about 30°C, above about 35°C, e.g., a temperature of about 37°C, which is approximately the average temperature of the human body.

In some embodiments, greater volumes of ECM proteins are used to produce thick three-dimensional gels for culturing cells. For example, thick gels may be useful in inducing cells to migrate from the surface to the interior of the gel. In some embodiments, this migratory behavior can serve as a model for tumor cell metastasis. In some embodiments, the culture medium comprises a layer with a thickness between about 1.0 mm and about 2.0 mm, such as about 1.5 mm or about 1.8 mm. The amount of culture medium may also be expressed as the volume (V) in a well plate according to the relationship V = (πr²)h, wherein h is the thickness of the layer and r is the radius. In some embodiments, for example, the volume of culture medium may range from about 400 µl to about 800 µl, such as about 700 µl, with r = 11.05 mm.

In some embodiments, the at least one stimulus as disclosed herein comprises a protein kinase C (PKC) activator. PKC activators are known in the art and include, but are not limited to, bradykinin, phorbol esters such as phorbol 12-myristate 13-acetate (PMA), phorbol 12,13-dibutyrate (PDBu), phorbol 12,13-didecanoate (PDD), bombesin, cholecystokinin, thrombin, prostaglandin F2u and vasopressin. Other PKC activators include natural and unnatural diacylglycerols (DAG), including diacylglycerols with various fatty acids in the 1,2-sn configuration are active. In a specific embodiment, the DAG contains an unsaturated fatty acid. In one embodiment, the PKC activator is a macrocyclic lactone, including but is not limited to those in bryostatin compound class and neristatin compound class. In another embodiment, the PKC activator is a benzolactam. In a further embodiment, the PKC activator is a pyrrolidinone. In a specific embodiment, the macrocyclic lactone is bryostatin. In a more specific embodiment, the bryostatin is bryostatin-1, - 2, -3, -4, -5, -6, -7, -8, -9, -10, -11, -12, -13, -14, -15, -16, -17, or -18.

In some embodiments, the at least one stimulus as disclosed herein comprises analogs of bryostatin. Analogs of bryostatin, commonly referred to as bryologs, are one particular class of PKC activators that are suitable for use in the methods of the present invention. Bryologs are structurally similar, but vary greatly in their affinity for PKC (from 0.25 nM to 10 uM). While bryostatin-1 has two pyran rings and one 6-membered cyclic acetal, in most bryologs one of the pyrans of bryostatin-1 is replaced with a second 6-membered acetal ring. This modification reduces the stability of bryologs, relative to bryostatin-1, for example, in both strong acid or base, but has little significance at physiological pH. Bryologs also have a lower molecular weight (ranging from about 600 to 755), as compared to bryostatin-1 (988), a property which facilitates transport across the blood-brain barrier. Various bryologs are described, for example, in U.S. Application No. 11/802,723, published as US 2008-0058396A1.

Other classes of PKC activators are polyunsaturated fatty acids ("PUFAs"). These compounds are essential components of the nervous system and have numerous health benefits. In general, PUFAs increase membrane fluidity, rapidly oxidize to highly bioactive products, produce a variety of inflammatory and hormonal effects, and are rapidly degraded and metabolized. The inflammatory effects and rapid metabolism is likely the result of their active carbon-carbon double bonds. These compounds may be potent activators of PKC, most likely by binding the PS site.

In one embodiment, the PUFA is chosen from linoleic acid (shown below).

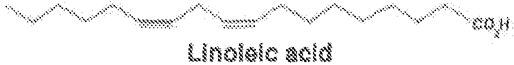

Another class of PKC activators are PUFA and MUFA derivatives, and cyclopropanated derivatives in particular. Certain cyclopropanated PUFAs, such as DCPLA (i.e., linoleic acid with cyclopropane at both double bonds), may be able to selectively activate PKC-ε. *See* Journal of Biological Chemistry, 2009, 284(50): 34514-34521; *see also* U.S. Patent Application Publication No. 2010/0022645 A1. Like their parent molecules, PUFA derivatives are thought to activate PKC by binding to the PS site.

Cyclopropanated fatty acids exhibit low toxicity and are readily imported into the brain where they exhibit a long half-life (t_{½}), Conversion of the double bonds into cyclopropane rings prevents oxidation and metabolism to inflammatory byproducts and creates a more rigid U-shaped 3D structure that may result in greater PKC activation. Moreover, this U-shape may result in greater isoform specificity. For example, cyclopropanated fatty acids may exhibit potent and selective activation of PKC-ε.

The Simmons-Smith cyclopropanation reaction is an efficient way of converting double bonds to cyclopropane groups. This reaction, acting through a carbenoid intermediate, preserves the *cis*-stereochemistry of the parent molecule. Thus, the PKC-activating properties are increased while metabolism into other molecules like bioreactive eicosanoids, thromboxanes, or prostaglandins is prevented.

One class of PKC-activating fatty acids are Omega-3 PUFA derivatives. In one embodiment, the Omega-3 PUFA derivatives are chosen from cyclopropanated docosahexaenoic acid, cyclopropanated eicosapentaenoic acid, cyclopropanated rumelenic acid, cyclopropanated parinaric acid, and cyclopropanated linolenic acid (CP3 form shown below).

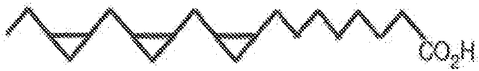

Another class of PKC-activating fatty acids are Omega-6 PUFA derivatives. In one embodiment, the Omega-6 PUFA derivatives are chosen from cyclopropanated linoleic acid ("DCPLA," CP2 form shown below), 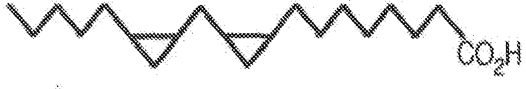 cyclopropanated arichidonic acid, cyclopropanated eicosadienoic acid, cyclopropanated dihomo-gamma-linolenic acid, cyclopropanated docosadienoic acid, cyclopropanated adrenic acid, cyclopropanated calendic acid, cyclopropanated docosapentaenoic acid, cyclopropanated jacaric acid, cyclopropanated pinolenic acid, cyclopropanated podocarpic acid, cyclopropanated tetracosatetranoic acid, and cyclopropanated tetracosapentaenoic acid.

Vernolic acid is a naturally occurring compound. However, it is an epoxyl derivative of linoleic acid and therefore, as used herein, is considered an Omega-6 PUFA derivative. In addition to vernolic acid, cyclopropanated vernolic acid (shown below) is an Omega-6 PUFA derivative.

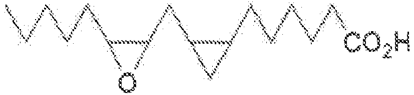

Another class of PKC-activating fatty acids are Omega-9 PUFA derivatives. In one embodiment, the Omega-9 PUFA derivatives are chosen from cyclopropanated eicosenoic acid, cyclopropanated mead acid, cyclopropanated erucic acid, and cyclopropanated nervonic acid.

Yet another class of PKC-activating fatty acids are monounsaturated fatty acid ("MUFA") derivatives. In one embodiment, the MUFA derivatives are chosen from cyclopropanated oleic acid (shown below), 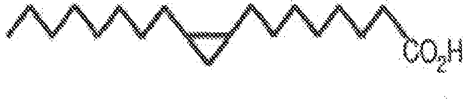 and cyclopropanated elaidic acid (shown below).

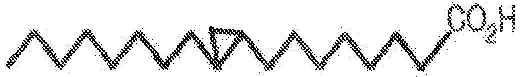

PKC-activating MUFA derivatives include epoxylated compounds such as trans-9,10-epoxystearic acid (shown below).

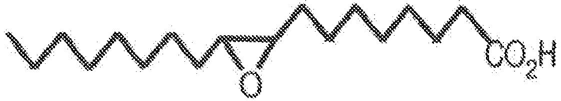

Another class of PKC-activating fatty acids are Omega-5 and Omega-7 PUFA derivatives. In one embodiment, the Omega-5 and Omega-7 PUFA derivatives are chosen from cyclopropanated rumenic acid, cyclopropanated alphaelostearic acid, cyclopropanated catalpic acid, and cyclopropanated punicic acid.

Another class of PKC activators are fatty acid alcohols and derivatives thereof, such as cyclopropanated PUFA and MUFA fatty alcohols. It is thought that these alcohols activate PKC by binding to the PS site. These alcohols can be derived from different classes of fatty acids.

In one embodiment, the PKC-activating fatty alcohols are derived from Omega-3 PUFAs, Omega-6 PUFAs, Omega-9 PUFAs, and MUFAs, especially the fatty acids noted above. In one embodiment, the fatty alcohol is chosen from cyclopropanated linolenyl alcohol (CP3 form shown below), 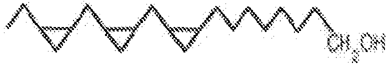 cyclopropanated linoleyl alcohol (CP2 form shown below), 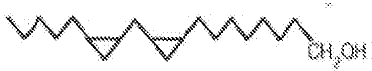 cyclopropanated elaidic alcohol (shown below), 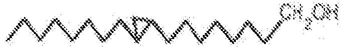 cyclopropanated DCPLA alcohol, and cyclopropanated oleyl alcohol.

Another class of PKC activators are fatty acid esters and derivatives thereof, such as cyclopropanated PUFA and MUFA fatty esters. In one embodiment, the cyclopropanated fatty esters are derived from Omega-3 PUFAs, Omega-6 PUFAs, Omega-9 PUFAs, MUFAs, Omega-5 PUFAs, and Omega-7 PUFAs. These compounds are thought to activate PKC through binding on the PS site. One advantage of such esters is that they are generally considered to be more stable that their free acid counterparts.

In one embodiment, the PKC-activating fatty acid esters derived from Omega-3 PUFAs are chosen from cyclopropanated eicosapentaenoic acid methyl ester (CP5 form shown below)

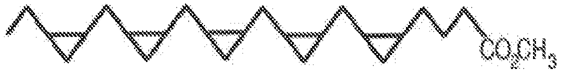

and cyclopropanated linolenic acid methyl ester (CP3 form shown below).

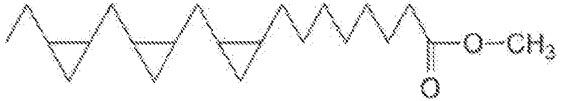

In another embodiment, the Omega-3 PUFA esters are chosen from esters of DHA-CP6 and aliphatic and aromatic alcohols. In one embodiment, the ester is cyclopropanated docosahexaenoic acid methyl ester (CP6 form shown below). 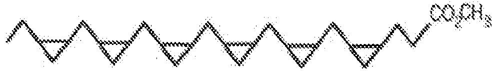 DHA-CP6, in fact, has been shown to be effective at a concentration of 10 nM. *See, e.g.,* U.S Patent Application Publication No. 2010/0022645.

In one embodiment, PKC-activating fatty esters derived from Omega-6 PUFAs are chosen from cyclopropanated arachidonic acid methyl ester (CP4 form shown below), 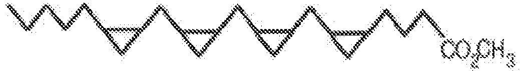 cyclopropanated vernolic acid methyl ester (CP1 form shown below), and 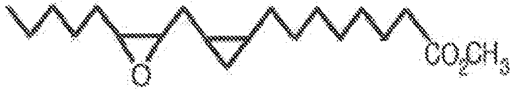 vernolic acid methyl ester (shown below).

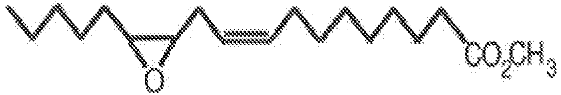

One particularly interesting class of esters are derivatives of DCPLA (CP6-linoleic acid). In one embodiment, the ester of DCPLA is an alkyl ester. The alkyl group, in one embodiment, may be chosen from methyl, ethyl, propyl (e.g., isopropyl), and butyl (e.g., tert-butyl) esters. DCPLA in the methyl ester form ("DCPLA-ME") is shown below.

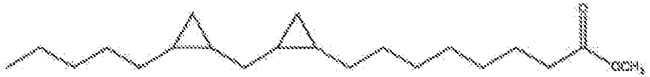

In another embodiment, the esters of DCPLA are derived from a benzyl alcohol (unsubstituted benzyl alcohol ester shown below). In yet another embodiment, the esters of DCPLA are derived from aromatic alcohols such as phenols used as antioxidants and natural phenols with pro-learning ability. Some specific examples include estradiol, butylated hydroxytoluene, resveratrol, polyhydroxylated aromatic compounds, and curcumin.

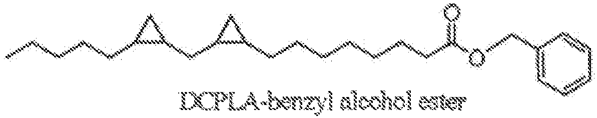

Another class of PKC activators is fatty esters derived from cyclopropanated MUFAs. In one embodiment, the cyclopropanated MUFA ester is chosen from cyclopropanated elaidic acid methyl ester (shown below), 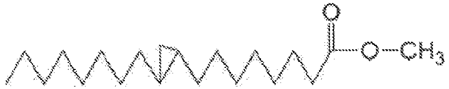 and cyclopropanated oleic acid methyl ester (shown below).

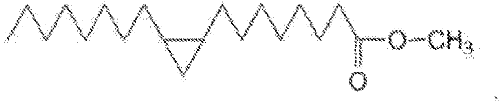

Another class of PKC activators are sulfates and phosphates derived from PUFAs, MUFAs, and their derivatives. In one embodiment, the sulfate is chosen from DCPLA sulfate and DHA sulfate (CP6 form shown below).

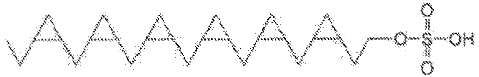

in one embodiment, the phosphate is chosen from DCPLA phosphate and DHA phosphate (CP6 form shown below).

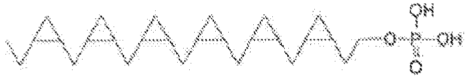

Further embodiments of PUFA and MUFA derivatives are disclosed in U.S. Patent No. 8,163,800.

In some embodiments, the at least one stimulus as disclosed herein comprises oligomeric Aβ (amylosheriods, ASPDs). For example, oligomeric Aβ can have a molecular weight of >100 kDa. These oligomers were reported to be highly toxic and had similarities to those found in the AD brain (Nouguchi et al., 2009).

In some embodiments, the at least one stimulus as disclosed herein comprises an agent. The agent includes, but is not limited to, bradykinin, insulin, phobol esters, lysophosphatidylcholine, lipopolysaccharide, anthracycline dannorubicin, and vanadyl sulfate.

### Gene Expression Profiling

Gene expression can be measured by both low-throughput methods such as Northern Blotting, *in situ* hybridization, reverse transcription quantitative polymerase chain reaction (RVQPCR), and real time PCR, and high-throughput methods such as microarrays and SAGE to detect differential gene expression. In one embodiment, detection is conducted using automatic, computerized equipment in a high-throughput setting, such as microarray technology.

In one embodiment, the method of the present disclosure provides detecting the gene transcript such as mRNA, including microRNA, cDNA or cRNA. The transcript can be from both coding and non-coding regions of the gene. The transcript can be detected *in situ* in the cell or in purified form extracted from the cell. In a specific embodiment, the nucleic acid is isolated and purified from the cell and then used in the gene expression assay.

In another embodiment, the method of the present disclosure provides detecting the protein product, or portion thereof, expressed from a gene transcript. Protein-based assays include low-throughput methods such as Western blotting and ELISA, and high throughput protein microarrays.

In a further embodiment, the method of the present disclosure further comprises detecting the activity or activation state of the detected protein product, such as the phosphorylation of given protein.

In one embodiment, gene transcripts *(e.g*., cDNAs) from two different cells are hybridized to the binding sites of known gene transcripts on a microarray, one which is the test cell that has been stimulated with at least one stimulus and another the control cell, preferably of the same cell type, which has been stimulated with at least one stimulus, preferably the same stimulus. The nucleic acid derived from each of the two cell types are differently labeled so that they can be distinguished. Use of microarrays to evaluate differentially expressed transcripts is well known. See, *e.g.,* U.S. 6,973,388. This technique typically involves preparing or purchasing microarrays containing known cDNA transcripts, extracting and labeling RNA from test cells, hybridizing the test RNA to the array, detecting and visualizing signal, performing statistical analysis on the results, and, optionally, validating the microarray results using low-throughput techniques.

Pre-made cDNA microarrays are commercially available from *e.g.*, Affymetrix® (Santa Clara, CA), Agilent Technologies® (Santa Clara, CA) and AlphaGene® (Woburn, MA). These include whole genome arrays and targeted subsets of known genes.

In another embodiment, differential expression of genes is detected using serial analysis of gene expression (SAGE). SAGE quantitatively determines the amount of times a small portion of a specific mRNA transcript is expressed (a tag). The output of SAGE is a list of short sequence tags and the number of times it is observed. The major difference between microarray hybridization and serial analysis of gene expression (SAGE) techniques is that the latter does not require prior knowledge of the sequences to be analyzed; SAGE is a sequencing-based gene expression profiling technique.

In one embodiment, the cell sample demonstrates an observable difference in the level of expression of one or more genes compared with the level of expression of the same gene or genes in the control cells. For example, the differential expression is quantitative. In a further example, the level of gene expression detected in the test cells is about 1-fold, 2-fold, 5-fold, 10-fold, and 100-fold upregulated or downregulated compared to the control cells.

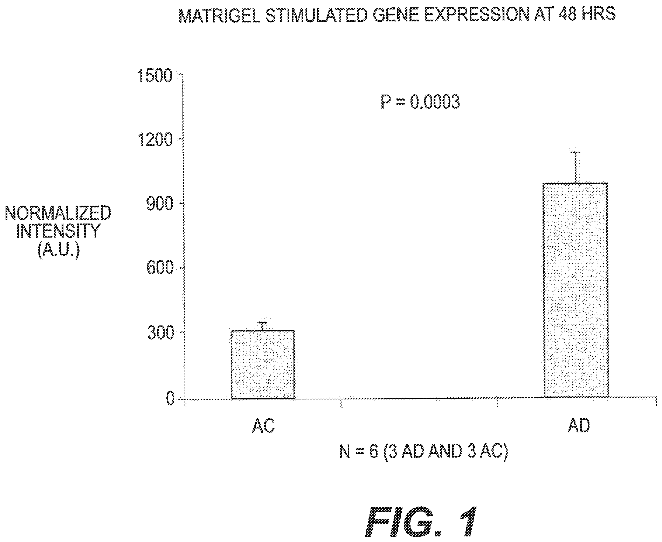

In some embodiments of the present disclosure, expression levels of the genes can be measured at about 1 hour, about 1.5 hours, about 2 hours, about 2.5 hours, about 3 hours, about 5 hours, about 8 hours, about 10 hours, about 12 hours, about 24 hours, about 36 hours, about 48 hours, about 60 hours, or even about 72 hours or more after culturing. For example, Figure 1 shows the expression level (Microarray data) of tumor necrosis factor receptor superfamily, member 19 (TNFRSF-19) gene in fibroblast cells from subjects with AD (left bar) and in fibroblast cells age-averaged control subjects ("AC") (right bar) at 48 hrs after stimulation with BD Matrigel™.

### Screening Methods for Therapeutics

In yet a further aspect, this disclosure relates to methods of screening therapeutic substances for the treatment or prevention of the neurodegenerative condition (AD) using the diagnostic tests described herein. According to this embodiment, compounds which reverse or improve the observed differences in gene expression described herein would be identified and selected as a substance potentially useful for the treatment or prevention of the neurodegenerative condition (AD).

In one embodiment, the screening method comprises the steps of contacting cells from a subject that has been diagnosed with AD with a test compound for a period of time, followed by contacting the cells with at least one stimulus as disclosed herein, and determining whether the test compound alters the differential expression of the genes identified according to the methods of the present disclosure towards levels observed in control cells from normal subjects.

In one embodiment, the cells contacted with the test compound are derived from a subject diagnosed with the neurodegenerative condition (AD) according to the methods of the present disclosure.

### Kits

This disclosure also relates to kits comprising products useful for carrying out the diagnostic methods as disclosed herein. The kits may also include instruments, buffers and storage containers necessary to perform one or more biopsies, such as punch skin biopsies. The kits can include high-density oligonucleotide arrays, reagents for use with the arrays, signal detection and array-processing instruments, gene expression databases and analysis and database management software. The kits may also contain instructions relating to the identification of differentially expressed genes used for the neurodegenerative condition (AD) diagnosis.

As stated previously, the kits may contain a single diagnostic test or any combination of the tests described herein. All of the differences disclosed herein between control and the neurodegenerative condition (AD) cells form the basis for the clinical tests and diagnostic kits for the neurodegenerative condition (AD) diagnosis, as well as the methods of screening compounds for treatment or prevention of the neurodegenerative condition (AD) disclosed herein.

### Combination Diagnostic Methods

It is contemplated that the diagnostic methods as disclosed herein may be used in combination with any other diagnostic methods. Exemplary methods include physical and neurological evaluation; biomarker detection; and structural (MRI, CT) and functional brain imaging (PET; FDG-PET).

As one example, the methods of the present disclosure can be used in combination with evaluating mutations in the genes known to be involved in Familial AD. Additional methods of diagnosing AD are described in U.S. patent 6,080,582 and 6,300,085 to Alkon et al., which methods detect the absence of potassium ion channels in the cells of an AD patient, differences in intracellular calcium ion concentration in AD and non-AD cells in response to potassium channel blockers specific for the potassium ion channel that is absent in the cells of an AD patient, and differences between AD and non-AD cells in response to activators of intracellular calcium release such as activators of inositol-1,4,5-trisphosphate (IP3). Additional diagnostic methods are described in application publication number WO2007/047029 to Alkon et al. directed to diagnosing AD in a subject by detecting alterations in the ratio of specific phosphorylated MAP kinase proteins (Erk1/Erk 2) in cells after stimulation with a PKC activator. See also, Zhao et al., Neurobiol. Dis. 2002 Oct; 11 (I): 166-83.

### EXAMPLES

### EXAMPLE 1

Three AD and samples of three age-matched control (AC) skin fibroblasts were used in this gene expression study. The fibroblasts from age-matched controls as well as non-AD dementia patients form small and higher number colonies of cells after 48 hrs, whereas fibroblasts from AD patients form large colonies that are few in number. A number of genes, which are listed in Tables 1 and 2, were determined to be up or down regulated in AD cells upon stimulation by BD Matrigel™. Figure 1 shows the expression level (Microarray data) of tumor necrosis factor receptor superfamily, member 19 (TNFRSF-19) gene in fibroblast cells from subjects with AD (left bar) and in fibroblast cells age-averaged control subjects ("AC") (right bar) at 48 hrs after stimulation with BD Matrigel™. As shown, the TNFRSF-19 gene is stimulated for AD cases compared to AC cases.

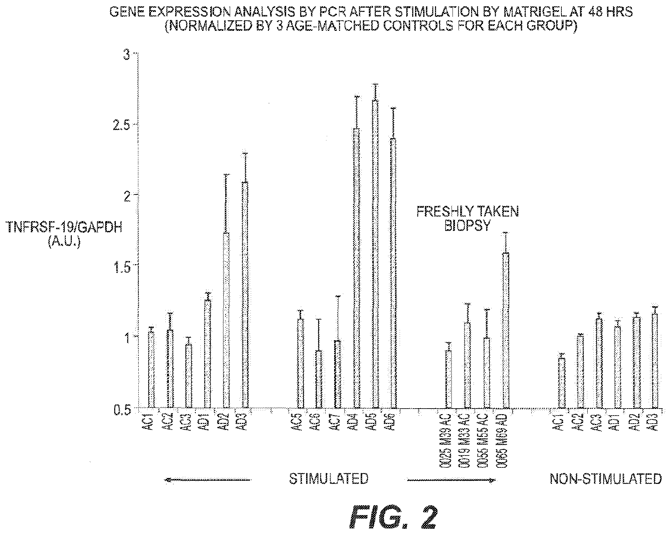

Figure 2 shows the expression levels (by PCR analysis) of the TNFRSF-19 gene in AD and AC subjects, stimulated by BD Matrigel™ for 48 hours or absent of stimulation by BD Matrigel™. The data was normalized by 3 age-matched controls for each group. The bars denoted by AC1 through AC6 and the bars denoted by AD1 through AD6 represent data obtained from non-freshly taken cells from the AC subjects or AD subjects. The bars denoted by 0025 M39 AC, 0019 M33 AC, 0055 M55 AC, and 0065 M69 AD represent data obtained from freshly taken biopsy fibroblast cells.

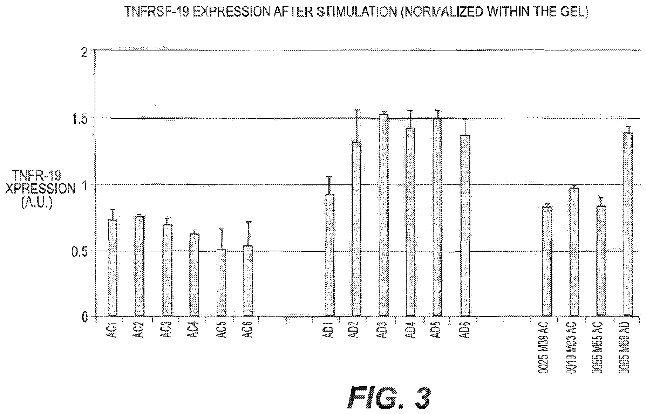

Figure 3 shows the expression levels (by PCR analysis) of the TNFRSF-19 gene in AD and AC subjects, stimulated by BD Matrigel™ for 48 hours. The data was normalized by normalized by each gel by PCR. The bars denoted by AC1 through AC6 and the bars denoted by AD1 through AD6 represent data obtained from non-freshly taken cells from the AC subjects or AD subjects. The bars denoted by 0025 M39 AC, 0019 M33 AC, 0055 M55 AC, and 0065 M69 AD represent data obtained from freshly taken biopsy fibroblast cells.

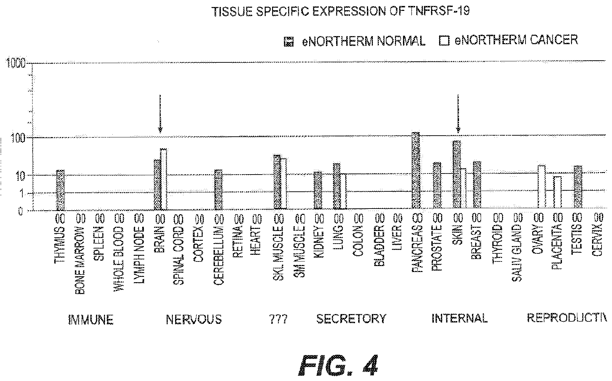

Figure 4 shows tissue specific expressions of the TNFRSF-19 gene in normal and cancer cells.

**BD Matrigel™ basement membrane preparation:** The BD Matrigel Matrix Growth Factor Reduced (BD Biosciences) was be thawed at 4°C on ice 30 min. before use. All pipettes, tips, and 12 well culture plates were be pre-cooled to 4°C before use. The BD Matrigel™ Matrix Growth Factor Reduced was mixed to homogeneity using cooled pipettes. No solid aggregates of the gel should be included within the mixture. 12 well culture plates were kept on ice for 30 min. prior to use and 700 µL of BD Matrigel™ Matrix Growth Factor Reduced per well will be added. The homogeneity of the gel on the surface of the cell culture plates was verified under the inverted microscope, and any bubble should be avoided. The 12-well plates were placed at 37°C for 30 minutes. The skin fibroblasts cell suspensions were added on top of BD Matrigel™ Matrix Growth Factor Reduced. The density of cells was adjusted to -50 cells/mm3.

**Recovery of Cells from BD Matrigel™ Matrix:** Cellular aggregates were recovered as follows: BD Cell Recovery Solution (BD Biosciences) was used to recover cells from BD Matrigel™ Matrix. First the cell culture medium was removed and washed the layer of cells on the BD Matrigel™ matrix three times with cold PBS. 2 mL of the recovery solution was per 35 mm dish. The cellular aggregates/gel layer was scraped into an ice-cold 50 ml conical tube sitting on ice. To recover all material from the dish was rinsed one time with 2mL of BD Cell Recovery Solution and was transferred to the tube. The BD Matrigel™ was completely dissolved by rocking the tube several time back and forth and kept on ice for 1 hour or until the BD Matrigel™ has complete dissolved. After about 30 minutes on ice, the cells were settled to the bottom of the tube indicating that the gel has been dissolved. The cellular aggregates were collected as a pellet at the bottom of the tube, by centrifuging at 200 - 300 xG for 5 minutes at 4°C. The cell pellet was washed by gentle resuspended in ice cold PBS and by centrifuging at same condition. RNA was isolated from the cell aggregates according to standard

**Gene Expression Profiling:** The microarray analysis was performed using the Human Whole Genome OneArray® v5 (Phalanx Biotech, Palo Alto, CA). RNA quality and integrity were determined utilizing an Agilent 2100 Bioanalyzer (Agilent Technologies, Palo Alto, CA, USA) and absorbance at A260/A280. Only high quality RNA, having a RIN of >7.0, and an A260/280 absorbance ratio of >1.8, was utilized for further experimentation. RNA was converted to double-stranded cDNA and amplified using in vitro transcription that included amino-allyl UTP, and the aRNA product was subsequently conjugated with Cy5™ NHS ester (GEH Lifesciences). Fragmented aRNA was hybridized at 50 °C overnight using the HybBag mixing system with 1X OneArray Hybridization Buffer (Phalanx Biotech), 0.01 mg/ml sheared salmon sperm DNA (Promega, Madison, WI, USA), at a concentration of 0.025 mg/ml labeled target.

After hybridization, the arrays were washed according to the OneArray protocol. Raw intensity signals for each microarray were captured using a Molecular Dynamics™ Axon 4100A scanner, measured using GenePixPro™ Software, and stored in GPR format. The data from all microarrays in each experimental set was then passed to Rosetta Resolver (Rosetta Biosoftware) for analysis. Testing was performed by combining technical replicates and performing statistical analyses using Rosetta Resolver's proprietary modeling techniques.

**Results:** All differentially expressed genes after aggregate formation at 48 hrs of incubation on BD Matrigel™ Matrix are tabulated in Tables 1 and 2.

**Table 1 Differentially expressed activated genes after stimulation**

| **Gene symbol** | **Fold change AD/AC** | ***P-value** | **Description** |
|---|---|---|---|
| EFEMP1 | 6.917 | 0.00035 | EGF-containing fibulin-like extracellular matrix protein 1 |
| #BDNF | 7.831 | 0.00007 | Brain-derived neurotrophic factor |
| FGF18 | 3.067 | 0.00222 | Fibroblast growth factor 18 |
| IGFBP5 | 9.681 | <0.000003 | Insulin-like growth factor binding protein 5 |
| HAS1 | 8.085 | 0.00003 | Hyaluronan synthase 1 |
| #CDH2 | 3.428 | 0.000001 | Cadherin 2, type 1, N-cadherin (neuronal) |
| CAPG | 3.056 | 0.007074 | Capping protein (actin filament), gelsolin-like |
| MMP12 | 2.798 | 0.036324 | Matrix metallopeptidase 12 (macrophage elastase) |
| #MAPK1 | 3.175 | 0.000007 | Mitogen-activated protein kinase 1 |
| TNFRSF19 | 3.222 | 0.000314 | Tumor necrosis factor receptor superfamily, member 19 |
| PPAPDC1A | 3.911 | 0.0229 | Phosphatidic acid phosphatase type 2 domain containing 1A |
| DUSP2 | 6.720 | <0.0000001 | Dual specificity phosphatase 2 |
| CRIP2 | 3.923 | 0.00039 | Cysteine-rich protein 2 |
| PPP1CB | 4.474 | 0.005376 | Protein phosphatase 1, catalytic subunit, beta isozyme |
| EDNRB | 4.855 | <0.00000018 | Endothelin receptor type B |

| | | | |
|---|---|---|---|
| # Genes as shown in Table 1 are related to Erk. | | | |

**Table 2 Differentially expressed de-activated genes after stimulation**

| **Gene symbol** | **Fold change AD/AC** | ***P-value** | **Description** |
|---|---|---|---|
| EGR2 | 0.385 | 0.000328 | Early growth response 2 |
| MAP2 | 0.294 | 0.015971 | Microtubule-associated protein 2 |
| HLA-C | 0.052 | <0.000001 | Major histocompatibility complex, class I, C |
| CADM1 | 0.535 | <0.000001 | Cell adhesion molecule 1 |
| COL23A1 | 0.161 | <0.000001 | Collagen, type XXIII, alpha 1 |
| BDKRB2 | 0.682 | 0.000382 | Bradykinin receptor B2 |
| APOE | | <0.000001 | Apolipoprotein E |
| NRG3 | 0.054 | <0.000001 | Neuregulin 3 |

| | | | |
|---|---|---|---|
| * P values as shown in Tables 1 and 2 are calculated from 3 AD and 3 AC cases. | | | |

**Table 3 Composition of BD Matrigel™ and its relation to signal transduction mechanisms in AD**

| Components | Subcomponents | Signal transduction pathways | Relation to AD pathways | References |
|---|---|---|---|---|
| Growth factors | TGFβs | Multifunctional cytokines. Work as chemotactic for lymphocytes, monocytes, neutrophils and fibroblasts. Induce angiogenesis. Extracellular matrix production | Regulation of APP synthesis and processing, plaque formation, astroglial and microglial activation, and neuronal apoptosis. Increase low density lipoprotein receptor-related protein expressions. Increase intraneuronal Aβ accumulation, toxicity. | Tseng, et al., (2004). FEBS Lett. 562: 71-78. Harris-White, et al., (2004), J. Neurosci. Res. 77: 217- 228. |
| | FGF | FGF treatment modulates ECM molecule production and gene expression | APP regulates EGF receptor expression | Bellucci et al., (2007), Mol. Med. 13: 542-550. |
| | PDGF | Platelet-derived growth factor | PDGF regulates the β-, γ-secretase-mediated cleavage of APP | Gianni et al., (2004), J. Bio. Chem. 278: 9290-9297. |
| | IGF | IGF signaling pathway regulates metabolism, cellular growth, and survival. | IGF protects cells from apoptosis in AD pathogenesis. | Niikura, T,. J. Neurosc. (2001), 21: 1902-1910. |
| Proteases | 72 kDa MMP-2 (Gelatinase A) | 72 kDa proteolytic enzyme interacts with cell surface lectins involved in cell-cell and cell-matrix and metastasis. | | |
| | | | *In vitro* degradation of Aβ | Roher et al., (1994), Biochem. Biophys .Res. Commun. 205:1755-1761. |
| | 92 kDa MMP-9 (Progelatinase B) | Releases from astrocytes, neurons, microglia, leukocytes and macrophages. Degrades ECM components including collagen, gelatin, fibronectin, laminin, elastin and proteoglycans, etc. | MMP-9 is found close to extracellular amyloid plaques. MMP-9 is elevated in the plasma of AD patients | Lorenzl et al., (2003), Neurochem. Int., 43: 191-196 |
| | urUokinase | | | |
| | Tissue type plasminoge n activator (tPA) | tPA dissolves blood clots and it has been used for ischemia prevention. | Aβ binds tPA and cause increased plasmin production | Kranenburg, et al., (2005), Neurosci.131:877-886 |
| Abundant components | Laminin | Laminins are a major component of ECM beside collagen IV and proteoglycans. | Promote neurite outgrowth. Cell adhesion activity by binding with the integrin receptor. Inhibition of Aβ fibril formation and reduction of Aβ-induced cytotoxicity. | Drouet, et al., (1999), J. Neurochem.:73, 742-749. Morgan et al., (2002), Peptides 23:1229-1240. |
| | Collagen type IV | A structural protein an important ECM component. | Collagen can cause a decrease in secretion and accumulation of APP | Kiuchi, et al., (2002), Life Sci. 70:1555-1564. |
| | Heparan sulfate proteoglyca n | Bind to APP Capable of binding ECM | Down regulated in LOAD fibroblasts | Ca ceres and Brandan, (1997), J. Cellular Biochem. 65:145-158 |
| | | molecules via their covalently attached heparan sulfate chains | | |
| | Nidogen/ent acin | A major component of basement membrane. Perlecan binds to nidogens, laminin/nidogen complex, fibronectin, fibulin-2 and heparin | Not yet established | |
| Other components | Clusterin | Interacts with Aβ, regulates cholesterol and lipid metabolism of brain which is disturbed in AD. | ApoeJ (clusterin) increases during AD | Nuutinen, et al., (2009), Brain Res. Rev. 62:89-104. |

**Table 4 Gene Symbol Report for TNFRSF19**

| **Approved Symbol** | Approved Name | Nucleotide Sequences | Synonyms | Chromosome |
|---|---|---|---|---|
| TNFRSF19 | Tumor necrosis factor receptor superfamily, member 19 | GenBank:AB040434 E MBL DDBJ **C** RefSeq:NM_ 001204458 **D** CCDS:CCDS9301.1 **C** Vega:OTTHUMG00000016568 **C** | TAJ-alpha, TROY, TAJ, TRADE | 13q12.11-q12.3 |

Other embodiments of the invention will be apparent to those skilled in the art from consideration of the specification and practice of the invention disclosed herein. It is intended that the specification and examples be considered as exemplary only.

## Claims

1. A method of diagnosing Alzheimer's disease in a human subject in need thereof comprising:
(a) contacting a cell sample from the subject with at least one stimulus, wherein the at least one stimulus is chosen from the group consisting of a polysaccharide mixture comprising at least one basement membrane protein a protein kinase C activator, an Aβ oligomer, or combinations thereof and wherein the cell sample is a fibroblast cell sample or a lymphocyte cell sample; and
(b) detecting the expression of at least one gene in the cell sample, wherein the at least one gene is selected from the group consisting of EFEMP1, BDNF, FGF18, IGFBP5, HAS1, CDH2, CAPG, MMP12, MAPK1, TNFRSF19, PPAPDC1A, DUSP2, CRIP2, PPP1CB, EDNRB, EGR2, MAP2, HLA-C, CADM1, COL23A1, BDKRB2, APOE, and NRG3, wherein
the subject has Alzheimer's disease if the expression level(s) determined in step (b) for one or more gene(s) selected from the group consisting of EFEMP1, BDNF, FGF18, 1GFBP5, HAS1, CDH2, CAPG, MMP12, MAPK1, TNFRSF19, PPAPDC1A, DUSP2, CRIP2, PPP1CB and EDNRB is greater than the expression level(s) of the same gene(s) in age-matched control cells that have been contacted with the same at least one stimulus, and/or
the subject has Alzheimer's disease if the expression level(s) determined in step (b) for one or more gene(s) selected from the group consisting of EGR2, MAP2, HLA-C, CADM1, COL23A1, BDKRB2, APOE and NRG3 is lower than the expression levels(s) of the same gene(s) in age-matched control cells that have been contacted with the same at least one stimulus.

2. The method of claim 1, wherein step (b) comprises determining in the cell sample the expression level(s) of one or more gene(s) selected from the group consisting of EFEMP1, BDNF, FGF18, IGFBP5, HAS1, CDH2, CAPG, MMP12, MAPK1, TNFRSF19, PPAPDC1A, DUSP2, CRIP2, PPP1CB and EDNRB, and one or more gene(s) selected from the group consisting of EGR2, MAP2, HLA-C, CADM1, COL23A1, BDKRB2, APOE and NRG3.

3. The method of claim 1, wherein the method comprises contacting a skin fibroblast cell sample from the subject with at least one stimulus.

4. The method of claim 1, wherein the method comprises contacting a lymphocyte cell sample from the subject with at least one stimulus.

5. The method of claim 1, wherein the gene expression level(s) are determined by a microarray.

6. The method of claim 1, wherein the Alzheimer's disease is chosen from sporadic Alzheimer's disease, early-stage Alzheimer's disease, and young-onset Alzheimer's disease.

7. The method of claim 1, wherein the diagnosis of the Alzheimer's disease is confirmed using one or more additional diagnostic methods.

8. The method of claim 1, wherein the at least one stimulus comprises two or more stimuli and the two or more stimuli are contacted with the cell sample simultaneously or sequentially.

9. The method of claim 1, wherein the stimulus is a protein kinase C-epsilon activator.

## Patentansprüche

1. Verfahren zur Diagnose der Alzheimer-Krankheit bei einem diesbezüglich bedürftigen menschlichen Patienten, umfassend:
(a) Inkontaktbringen einer Zellprobe des Patienten mit mindestens einem Stimulus, wobei der mindestens eine Stimulus ausgewählt wird aus der Gruppe bestehend aus einer Polysaccharidmischung, die mindestens ein Basalmembranprotein umfasst, einem Proteinkinase-C-Aktivator, einem Aß-Oligomer oder Kombinationen davon, und wobei die Zellprobe eine Fibroblastenzellprobe oder eine Lymphozytenzellprobe ist, und
(b) Nachweis der Expression von mindestens einem Gen in der Zellprobe, wobei das mindestens eine Gen ausgewählt wird aus der Gruppe bestehend aus EFEMP1, BDNF, FGF18, IGFBP5, HAS1, CDH2, CAPG, MMP12, MAPK1, TNFRSF19, PPAPDC1A, DUSP2, CRIP2, PPP1CB, EDNRB, EGR2, MAP2, HLA-C, CADM1, COL23A1, BDKRB2, APOE, und NRG3, wobei
der Patient die Alzheimer-Krankheit aufweist, wenn das/die Expressionsniveau/- s, das/die in Schritt (b) für ein oder mehrere Gen/-e ausgewählt aus der Gruppe bestehend aus EFEMP1, BDNF, FGF18, 1GFBP5, HAS1, CDH2, CAPG, MMP12, MAPK1, TNFRSF19, PPAPDC1A, DUSP2, CRIP2, PPP1 CB und EDNRB nachgewiesen wurde/-n, höher ist als das/die Expressionsniveau/-s des/derselben Gens/-e in Kontrollzellen gleichen Alters, die mit demselben mindestens einen Stimulus in Kontakt gebracht wurden, und/oder
der Patient die Alzheimer-Krankheit aufweist, wenn das/die Expressionsniveau/- s, das/die in Schritt (b) für ein oder mehrere Gen/-e ausgewählt aus der Gruppe bestehend aus EGR2, MAP2, HLA-C, CADM1, COL23A1, BDKRB2, APOE und NRG3 nachgewiesen wurde/-n, niedriger ist als das/die Expressionsniveau/-s des/derselben Gens/-e in Kontrollzellen gleichen Alters, die mit demselben mindestens einen Stimulus in Kontakt gebracht wurden.

2. Verfahren nach Anspruch 1, wobei Schritt (b) das Nachweisen des/der Expressionsniveaus von einem oder mehreren Gen/-en ausgewählt aus der Gruppe bestehend aus EFEMP1, BDNF, FGF18, IGFBP5, HAS1, CDH2, CAPG, MMP12, MAPK1, TNFRSF19, PPAPDC1A, DUSP2, CRIP2, PPP1CB und EDNRB und einem oder mehreren Gen/-en ausgewählt aus der Gruppe bestehend aus EGR2, MAP2, HLA-C, CADM1, COL23A1, BDKRB2, APOE und NRG3 umfasst.

3. Verfahren nach Anspruch 1, wobei das Verfahren das Inkontaktbringen einer Hautfibroblastenzellprobe des Patienten mit mindestens einem Stimulus umfasst.

4. Verfahren nach Anspruch 1, wobei das Verfahren das Inkontaktbringen einer Lymphozytenzellprobe des Patienten mit mindestens einem Stimulus umfasst.

5. Verfahren nach Anspruch 1, wobei das/die Gen-Expressionsniveau/-s durch ein Mikroarray nachgewiesen wird/werden.

6. Verfahren nach Anspruch 1, wobei die Alzheimer-Krankheit ausgewählt wird aus sporadischer Alzheimer-Krankheit, Alzheimer-Krankheit im Frühstadium und früh einsetzender Alzheimer-Krankheit.

7. Verfahren nach Anspruch 1, wobei die Diagnose der Alzheimer-Krankheit unter Verwendung einer oder mehrerer zusätzlicher Diagnoseverfahren bestätigt wird.

8. Verfahren nach Anspruch 1, wobei der mindestens eine Stimulus zwei oder mehr Stimuli umfasst und die zwei oder mehr Stimuli gleichzeitig oder nacheinander mit der Zellprobe in Kontakt gebracht werden.

9. Verfahren nach Anspruch 1, wobei der Stimulus ein Proteinkinase-C-Epsilonaktivator ist.

## Revendications

1. Un procédé de diagnostic de la maladie d'Alzheimer chez un sujet humain qui en a besoin, comprenant :
(a) le fait de mettre en contact un échantillon de cellules provenant du sujet avec au moins un stimulus, ledit au moins un stimulus étant choisi dans le groupe constitué d'un mélange de polysaccharides comprenant au moins une protéine de membrane basale, un activateur de protéine kinase C, un oligomère Aβ ou leurs combinaisons, et l'échantillon de cellules étant un échantillon de cellules fibroblastes ou un échantillon de cellules lymphocytaire ; et
(b) le fait de détecter l'expression d'au moins un gène dans l'échantillon de cellules, ledit au moins un gène étant choisi dans le groupe constitué par EFEMP1, BDNF, FGF18, IGFBP5, HAS1, CDH2, CAPG, MMP12, MAPK1, TNFRSF19, PPAPDC1A, DUSP2, CRIP2, PPP1CB, EDNRB, EGR2, MAP2, HLA-C, CADM1, COL23A1, BDKRB2, APOE et NRG3,
le sujet étant atteint de la maladie d'Alzheimer si le ou les niveaux d'expression déterminés à l'étape (b) pour un ou plusieurs gènes choisis dans le groupe constitué par EFEMP1, BDNF, FGF18, IGFBP5, HAS1, CDH2, CAPG, MMP12, MAPK1, TNFRSF19, PPAPDC1A, DUSP2, CRIP2, PPP1CB et EDNRB est supérieur au(x) niveau(s) d'expression du (des) même(s) gène(s) dans les cellules témoins appariées selon l'âge qui ont été en mises en contact avec le même au moins un stimulus, et/ou
le sujet ayant la maladie d'Alzheimer si le(s) niveau(s) d'expression déterminé à l'étape (b) pour un ou plusieurs gènes choisis dans le groupe constitué par EGR2, MAP2, HLA-C, CADM1, COL23A1, BDKRB2, APOE et NRG3 est inférieur aux niveaux d'expression du (des) même(s) gène(s) dans les cellules témoins appariés selon l'âge qui ont été mises en contact avec le même au moins un stimulus.

2. Le procédé selon la revendication 1, dans lequel l'étape (b) comprend le fait de déterminer, dans l'échantillon cellulaire, le ou les niveaux d'expression d'un ou plusieurs gènes choisis dans le groupe constitué par EFEMP1, BDNF, FGF18, IGFBP5, HAS1, CDH2, CAPG, MMP12, MAPK1, TNFRSF19, PPAPDC1A, DUSP2, CRIP2, PPP1CB et EDNRB, et un ou plusieurs gènes choisis dans le groupe comprenant EGR2, MAP2, HLA-C, CADM1, COL23A1, BDKRB2, APOE et NRG3.

3. Le procédé selon la revendication 1, dans lequel le procédé comprend le fait de mettre en contact un échantillon de cellules de fibroblastes de peau provenant du sujet avec au moins un stimulus.

4. Le procédé selon la revendication 1, dans lequel le procédé comprend le fait de mettre en contact un échantillon de cellules lymphocytaires provenant du sujet avec au moins un stimulus.

5. Le procédé selon la revendication 1, dans lequel le(s) niveau(x) d'expression génique(s) sont déterminés par un micro-réseau.

6. Le procédé selon la revendication 1, dans lequel la maladie d'Alzheimer est choisie parmi la maladie d'Alzheimer sporadique, la maladie d'Alzheimer à un stade précoce et la maladie d'Alzheimer à début précoce.

7. Le procédé selon la revendication 1, dans lequel le diagnostic de la maladie d'Alzheimer est confirmé en utilisant une ou plusieurs méthodes de diagnostic supplémentaires.

8. Le procédé selon la revendication 1, dans lequel ledit au moins un stimulus comprend deux stimuli ou plus et les deux stimuli ou plus sont mis en contact avec l'échantillon cellulaire simultanément ou séquentiellement.

9. Le procédé selon la revendication 1, dans lequel le stimulus est un activateur de protéine kinase C-epsilon.
